(19) Europäisches Patentamt
European Patent Office
Office européen des brevets

(11) Publication number: **0 415 103 B1**

# (12) EUROPEAN PATENT SPECIFICATION

(45) Date of publication of patent specification: **01.02.95**

(51) Int. Cl.[6]: **C07D 401/12**, C07D 401/14, C07D 409/14, A61K 31/44

(21) Application number: **90114661.3**

(22) Date of filing: **31.07.90**

The file contains technical information submitted after the application was filed and not included in this specification

## (54) 2,3-Dihydro-1-(pyridinylamino)-indoles, a process for their preparation and their use as medicaments.

(30) Priority: **02.08.89 US 388415**

(43) Date of publication of application:
**06.03.91 Bulletin 91/10**

(45) Publication of the grant of the patent:
**01.02.95 Bulletin 95/05**

(84) Designated Contracting States:
**AT BE CH DE DK ES FR GB GR IT LI LU NL SE**

(56) References cited:
**EP-A- 0 287 982**
**EP-A- 0 376 155**

(73) Proprietor: **HOECHST-ROUSSEL PHARMACEUTICALS INCORPORATED**
**Route 202-206 North**
**Somerville**
**New Jersey 08876 (US)**

(72) Inventor: **Effland, Richard Charles, Dr.**
**544 Rolling Hills Road**
**Bridgewater, NJ 08807 (US)**
Inventor: **Wettlaufer, David Gordon, Dr.**
**9 Chaucer Terrace**
**Phillipsburg, NJ 08865 (US)**
Inventor: **Klein, Joseph Thomas**
**1075 Route 202-206 North**
**Bridgewater, NJ 08807 (US)**

(74) Representative: **Losert, Wolfgang Dr. et al**
**HOECHST AKTIENGESELLSCHAFT,**
**Zentrale Patentabteilung,**
**Gebäude F 821**
**D-65926 Frankfurt am Main (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 415 103 B1

## Description

This invention relates to compounds of the formula (I)

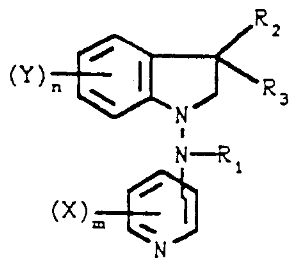

(I)

wherein $R_1$ is hydrogen, $C_1$-$C_6$-alkyl, alkenyl or alkynyl; $R_2$ and $R_3$ are independently hydrogen or $C_1$-$C_6$-alkyl, or taken together form a spiro-fused phenyl cycloalkane; X is halogen, $C_1$-$C_6$-alkyl or nitro and Y is $C_1$-$C_6$-alkyl; m and n are independently integers of 0 or 1, the pharmaceutically acceptable acid addition salts thereof and, where appropriate, the optical, geometrical and stereoisomers and racemic mixtures thereof.

Preferred embodiments of the invention are those of Compound I where $R_1$ is selected from hydrogen and $C_1$-$C_6$-alkyl, $R_2$ is selected from hydrogen and $C_1$-$C_6$-alkyl, and $R_3$ is selected from hydrogen and $C_1$-$C_6$-alkyl.

Most preferred embodiments of the invention are those of Compound I where $R_1$ is $C_1$-$C_6$-alkyl and $R_2$ and $R_3$ are hydrogen.

Throughout the specification and the appended claims, a given chemical formula or name shall encompass all optical and stereoisomers thereof and racemic mixtures where such isomers and mixtures exist.

In the above definition, the term "alkyl" refers to a straight or branched chain hydrocarbon containing no unsaturation, e.g., methyl, ethyl, isopropyl, t-butyl, neopentyl and n-hexyl; the term "alkylene" refers to a bivalent radical of the lower branched or unbranched alkyl group it is derived from having valence bonds from two terminal carbons thereof; e.g., methylen ($-CH_2-$), ethylene ($-CH_2CH_2-$), propylene ($-CH_2CH_2CH_2-$) and isopropylene

$$(CH_3\overset{|}{C}H\text{-}CH_2\text{-});$$

the term "alkoxy" refers to a monovalent substituent which consists of an alkyl group linked through an ether oxygen having its free valence bond from the ether oxygen, e.g., methoxy, ethoxy, propoxy, butoxy and pentoxy; and the term "halogen" refers to a member of the halogen family consisting of fluorine, chlorine, bromine and iodine.

EP-A-0 287 982 discloses N-pyridinyl-1H-indol-1-amines having memory enhancing, analgesic and/or antidepressant activities.

The compounds of the present invention are prepared in the following manner. The substituents are as defined above unless indicated otherwise.

Compound I of the invention is prepared through the reduction of 1,3-dihydro-1-(4-pyridinylamino)-2H-indol-2-one, of the formula

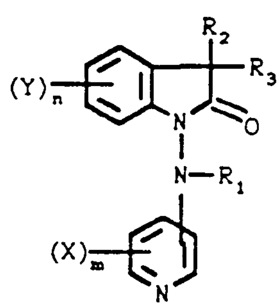

(II)

This reduction is typically carried out in the presence of a reducing agent, e.g., a borane-tetrahydrofuran complex or lithium aluminum hydride, in the presence of an ethereal solvent, e.g., tetrahydrofuran, and an inert atmosphere, e.g., nitrogen, etc., at a temperature of 0° to 25°C to reflux for 1/4 to 3 hours. Compound II is prepared by reacting compound III, a 1-aminooxindole of the formula

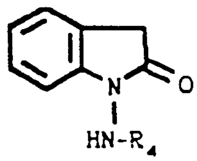

(III)

where $R_4$ is hydrogen or $C_1$-$C_6$-alkyl, with halopyridine hydrochloride. This reaction is typically conducted with compound III in solution with a $C_1$-$C_6$-alkanol or phenolic solvent, i.e., phenol, isopropanol or butanol, at a temperature of 80°C to 150°C for 1/2 to 24 hours. Compound III is typically synthesized utilizing procedures described in Baumgarten et al., J. Am. Chem. Soc. 82, 3977-82 (1960), which describes the formation of 1-aminooxindole by the reduction of 3-cinnolinol with zinc and $H_2SO_4$ and by thermal cyclization of O-hydrazinophenylacetic acid.

Compounds of the present invention are useful as analgesic agents due to their ability to alleviate pain in mammals. The activity of the compounds is demonstrated in the phenyl-para-quinone writhing assay in mice, a standard assay for analgesia [Proc. Soc. Exptl. Biol. Med., 95, 729 (1957)]. Presented in Table 1 is the analgesic effect of some of the compounds of the invention expressed either as the subcutaneous dose at which 50 % of the phenyl-paraquinone induced writing is inhibited in the animals, i.e., the $ED_{50}$ value, or as the % decrease in writhing at a given dose.

Table 1

| Compound (s.c.) | $ED_{50}$ or % Inhibition of Writhing |
|---|---|
| 2,3-Dihydro-N-(4-pyridinyl)-1H-indol-1-amine | 74% at 20 mg/kg |
| 2,3-Dihydro-N-propyl-N-(4-pyridinyl)-1H-indol-1-amine oxalate | 100% at 20 mg/kg |
| Aspirin (standard) | $ED_{50}$ = 32.8 mg/kg |

The analgesic relief of pain is achieved when the compounds of the invention are administered to a subject requiring such treatment at an effective oral, parenteral or intravenous dose of from 0.01 to 100 mg/kg of body weight per day. A preferred effective dose within this range is from about 10 to 50 mg/kg of body weight per day. A particularly preferred effective amount is about 30 mg/kg of body weight per day. It is to be understood, however, that for any particular subject, specific dosage regimens should be adjusted according to the individual need and the professional judgment of the person administering or supervising the administration of the compound. It is further to be understood that the dosages set forth herein are examples only and that they do not, to any extent, limit the scope or practice of the invention.

The compounds of the present invention are also useful in the treatment of various memory dysfunctions characterized by decreased cholinergic function as Alzheimer's Disease.

This utility is demonstrated in the Dark Avoidance Assay.

Dark Avoidance Assay

In this assay, mice are tested for their ability to remember an unpleasant stimulus for a period of 24 hours. A mouse is placed in a chamber that contains a dark compartment; a strong incandescent light drives it to the dark compartment, where an electric shock is administered through metal plates on the floor. The animal is removed from the testing apparatus and tested again, 24 hours later, for the ability to remember the electric shock.

If scopolamine, an anticholinergic that is known to cause memory impairment, is administered before an animal's initial exposure to the test chambers, the animal re-enters the dark compartment shortly after being placed in the test chamber 24 hours later. The effect of scopolamine is blocked by an active test compound, resulting in a greater interval before re-entry into the dark compartment.

The results for an active compound are expressed as the percent of a group of animals in which the effect of scopolamine is blocked, as manifested by an increased interval between being placed in the test

chamber and re-entering the dark compartment. The activity of some of the compounds of the instant invention in the assay are given below in Table 2.

Table 2

| Compound | Dose (mg/kg of body wt) | % of Animals With Scopolamine Induced Memory Deficit Reversal |
|---|---|---|
| 2,3-Dihydro-N-(4-pyridinyl)-1H-indol-1-amine | 0.3 mg/kg s.c. | 53% |
| Tacrine (standard) | 0.63 mg/kg s.c | 13% |
| Pilocarpine (standard) | 1.25 mg/kg s.c | 19% |

The compounds of the invention are also useful as anticonvulsants due to their anticonvulsant activity in mammals. Anticonvulsant activity is measured in the male mouse using the supramaximal electroshock (SES) assay described in Arch. Int. Pharmacodyn. 92:97-107, 1952. In this procedure, groups of male mice are used. Drugs are prepared using distilled water and, if insoluble, a surfactant is added. Control animals receive vehicle. Drugs are routinely administered interperitoneally (i.p.). The dosage volume is 10 ml/kg. A primary screen is given a 30 minute pretreat. The animals' eyes are placed across the output terminals of an A.C. shocker that delivers 206 volts rms for 300 msec. Electrode paste coats the animals' eyes at the point of contact with the terminals. A compound is considered to give protection if the mouse does not exhibit extensor tonus. Protection is expressed as normalized percent inhibition relative to vehicle control.

The anticonvulsant activity of some of the compounds is given below in Table 3.

Table 3

| Compound | % Inhibition |
|---|---|
| 2,3-Dihydro-N-(4-pyridinyl)-1H-indol-1-amine | -40% at 15 mg/kg i.p. |
| 2,3-Dihydro-N-propyl-N-(4-pyridinyl)-1H-indol-1-amine oxalate | -40% at 10 mg/kg i.p. |
| Phenobarbital (standard) i.p. | -50% at 8.4 mg/kg |

Effective amounts of the present invention may be administered to a subject by any one of various methods, for example, orally as in capsules or tablets, parenterally in the form of sterile solutions or suspensions, and in some cases intravenously in the form of sterile solutions. The compounds of the present invention, while effective themselves, may be formulated and administered in the form of their pharmaceutically acceptable addition salts for purposes of stability, convenience or crystallization, increased solubility and the like.

Preferred pharmaceutically acceptable addition salts include salts of inorganic acids such as hydrochloric, hydrobromic, sulfuric, nitric, phosphoric and perchloric acids; as well as organic acids such as tartaric, citric, acetic, succinic, maleic, fumaric, and oxalic acids.

The active compounds of the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 75% of the weight of the unit. The amount of compound present in such composition is such that a suitable dosage will be obtained. Preferred compositions and preparations according to the present invention are prepared so that an oral dosage unit form contains between 1.0-300 mgs of active compound.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel®, corn starch and the like; a lubricant such as magnesium stearate or Sterotex®; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as fatty oil. Other dosage unit forms may contain other various materials which

modify the physical form of the doseage unit, for example, as coatings. Thus tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of the aforesaid compound, but may be varied between 0.5 and about 30% of the weight thereof. The amount of active compound in such compositions is such that a suitable dosage will be obtained. preferred compositions and preparations according to the present invention are prepared so that a parenteral dosage unit contains between 0.5 to 100 mgs of active compound.

The solutions or suspensions may also include the following components; a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

Examples of the compounds of this invention include:

2,3-Dihydro-N-(3-nitro-4-pyridinyl)-1H-indol-1-amine;
2,3 -Dihydro-3-methyl-N-propyl-N-(4-pyridinyl)-1H-indol-1-amine;
N-Ethyl-2,3-dihydro-3,3-dimethyl-N-(3-methyl-4-pyridinyl)-1H-indol-1-amine;
N-Butyl-N-(3-fluoro-4-pyridinyl)-2,3-dihydro-5-methyl-3-phenylmethyl-1H-indol-1-amine;
N-(3-Amino-4-pyridinyl)-3,3-diethyl-2,3-dihydro-1H-indol-1-amine;
2,3-Dihydro-N-propyl-N-(3-propyl-4-pyridinyl)-1H-indol-1-amine;
2,3-Dihydro-6-methyl-N-(3-nitro-4-pyridinyl)-N-(2-propenyl)-1H-indol-1-amine;
2,3-Dihydro-N-methyl-N-(3-methyl-4-pyridinyl)-3-phenyl-methyl-1H-indol-1-amine;
2,3-Dihydro-N-(2-propynyl)-N-(4-pyridinyl)-1H-indol-1-amine;
N-(3-Amino-4-pyridinyl)-3,3-diethyl-2,3-dihydro-N-propyl-1H-indol-1-amine;
N-(3-Fluoro-4-pyridinyl)-2,3-dihydro-3-methyl-1H-indol-1-amine;
N-(3-Amino-4-pyridinyl)-N-butyl-2,3-dihydro-3-phenylethyl-1H-indol-1-amine;
2,3-Dihydro-N-(4-nitro-3-pyridinyl)-1H-indol-1-amine;
N-(4-Fluoro-3-pyridinyl)-2,3-dihydro-3-methyl-1H-indol-1-amine;
2,3-Dihydro-N-propyl-N-(3-pyridinyl)-1H-indol-1-amine.
1,2′,3,3′-Tetrahydro-N-(4-pyridinyl)-spiro[2H-indene-2,3′-[3H] indol]-1′-amine

The following examples are for illustrative purposes. All temperatures are given in degrees centigrade (°C) unless otherwise designated.

## EXAMPLE 1

### 2,3-Dihydro-N-(4-pyridinyl)-1H-indol-1-amine

To a stirred solution consisting of 1,3-dihydro-1-(4-pyridinylamino)-2H-indol-2-one (5.00 g) and THF (500 ml) was added borane-THF complex (66.7 ml, 1M concentration). The flask was fitted with reflux condensor and nitrogen inlet. The reaction mixture was heated at reflux 45 minutes followed by cooling to room temperature and concentrated. To the resulting foam was slowly added 5% aqueous hydrochloric acid (500 ml) and methanol (500 ml) after which time the reaction mixture was heated at reflux for 3 hours. The solution was cooled to room temperature and neutralized with saturated aqueous sodium bicarbonate. After gas evolution had ceased, the aqueous layer was extracted four times with ethyl acetate and once with ether. The combined organic layers were washed with water, brine, and dried ($K_2CO_3$). Filtration and concentration gave the crude product.

Purification via preparative high performance liquid chromatography (HPLC) afforded 2.20 g (47%) of 2,3-dihydro-N-(4-pyridinyl)-1H-indol-1-amine, a solid, m.p. 125-126.5°C.

| ANALYSIS: | | | |
|---|---|---|---|
| Calculated for $C_{13}H_{13}N_3$: | 73.91%C | 6.20%H | 19.89%N |
| Found: | 73.60%C | 6.23%H | 20.01%N |

EXAMPLE 2

2,3-Dihydro-N-propyl-N-(4-pyridinyl)-1H-indol-1-amine oxalate

To a solution consisting of 2,3-dihydro-N-(4-pyridinyl)-1H-indol-1-amine (2.20 g) and DMF (95 ml), cooled to 0 °C under nitrogen, was added sodium hydride (0.27 g). Stirring was continued at 0 °C for 50 minutes at which time bromopropane (1.05 ml) was added dropwise. After 2 hours, the reaction mixture was poured into water and ethyl acetate. The layers were separated and the aqueous layer extracted twice with ethyl acetate and once with ether. The combined organic layers were washed with brine and dried ($K_2CO_3$). Filtration and concentration gave the crude product.

Purification via flash column chromatography afforded 1.80 g (68%) of 2,3-dihydro-N-propyl-N-(4-pyridinyl)-1H-indol-1-amine as a foam. The oxalate was prepared with 0.97 eq. oxalic acids in absolute ethanol and ether. The resulting solid was washed with ether, m.p. 165.5-166.5 °C.

| ANALYSIS: | | | |
|---|---|---|---|
| Calculated for $C_{18}H_{21}N_3O_4$: | 62.96%C | 6.16%H | 12.24%N |
| Found: | 62.92%C | 6.10%H | 12.21%N |

## Claims

**Claims for the following Contracting States : AT, BE, CH, DE, DK, FR, GB, IT, LI, LU, NL, SE**

**1.** A compound of the formula I

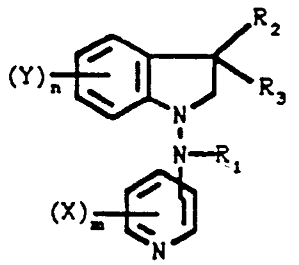

(I)

wherein $R_1$ is hydrogen, $C_1$-$C_6$-alkyl, alkenyl or alkynyl; $R_2$ and $R_3$ are independently hydrogen or $C_1$-$C_6$-alkyl, or taken together form a spiro-fused phenyl cycloalkane; X is halogen, $C_1$-$C_6$-alkyl or nitro and Y is $C_1$-$C_6$-alkyl; m and n are independently integers of 0 or 1, the pharmaceutically acceptable acid addition salts thereof and where applicable, the optical, geometrical and stereosimers and racemic mixtures thereof.

**2.** A compound as defined in Claim 1 wherein $R_1$ is hydrogen or $C_1$-$C_6$-alkyl, $R_2$ is hydrogen or $C_1$-$C_6$-alkyl and $R_3$ is hydrogen or $C_1$-$C_6$-alkyl.

**3.** A compound is defined in Claim 2 wherein $R_1$ is hydrogen or $C_1$-$C_6$-alkyl and $R_2$ and $R_3$ are hydrogen.

**4.** The compound as defined in Claim 3 which is 2,3-dihydro-N-(4-pyridinyl)-1H-indol-1-amine, or a pharmaceutically acceptable acid addition salt thereof.

**5.** The compound as defined in Claim 3 which is 2,3-dihydro-N-propyl-N-(4-pyridinyl)-1H-indol-amine, or a pharmaceutically acceptable acid addition salt thereof.

6. A pharmaceutically composition which comprises as the active ingredient a compound as defined in Claim 1 and a suitable carrier therefor.

7. Use of a compound as defined in claim 1 for the preparation of a medicament having analgesic, anticonvulsant and/or memory enhancing activity.

8. A process for the preparation of a compound as defined in Claim 1, which comprises reducing a compound of the formula II

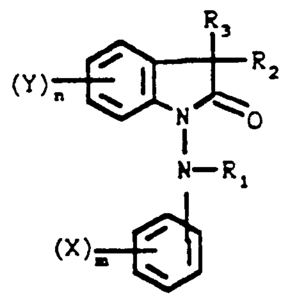

(II)

wherein $R_1$, $R_2$, $R_3$, X, Y, m and n are defined according to claim 1.

**Claims for the following Contracting States : ES, GR**

1. A process for the preparation of a compound of the formula I

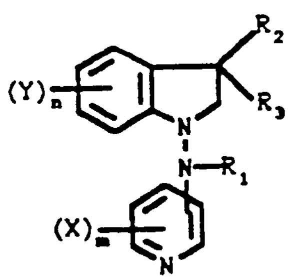

(I)

wherein $R_1$ is hydrogen, $C_1$-$C_6$-alkyl, alkenyl or alkynyl; $R_2$ and $R_3$ are independently hydrogen or $C_1$-$C_6$-alkyl, or taken together form a spiro-fused phenyl cycloalkane; X is halogen, $C_1$-$C_6$-alkyl or nitro and Y is $C_1$-$C_6$-alkyl; m and n are independently integers of 0 or 1, the pharmaceutically acceptable acid addition salts thereof and where applicable, the optical, geometrical and stereosimers and racemic mixtures thereof, which comprises reducing a compound of the formula II

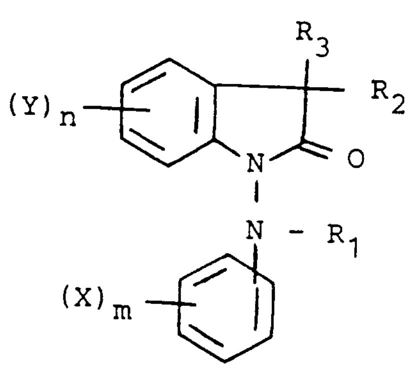

(II)

wherein $R_1$, $R_2$, $R_3$, X, Y, m and n are as previously defined.

2. A process as defined in claim 1 wherein the reduction is carried out with a borane-tetrahydrofuran complex or lithium aluminium hydride.

3. A process as defined in claim 1 wherein $R_1$ is hydrogen or $C_1$-$C_6$-alkyl, $R_2$ is hydrogen or $C_1$-$C_6$-alkyl and $R_3$ is hydrogen or $C_1$-$C_6$-alkyl.

4. A process as defined in claim 1 wherein $R_1$ is hydrogen or $C_1$-$C_6$-alkyl and $R_2$ and $R_3$ are hydrogen.

5. The process as defined in claim 1 wherein 2,3-dihydro-N-(4-pyridinyl)-1H-indol-1-amine or a pharmaceutically acceptable acid addition salt thereof is prepared.

6. The process as defined in claim 1 wherein 2,3-dihydro-N-propyl-N-(4-pyridinyl)-1H-indol-amino or a pharmaceutically acceptable acid addition salt thereof is prepared.

7. Use of a compound as defined in claim 1 for the preparation of a medicament having analgesic, anticonvulsant and/or memory enhancing activity.

**Patentansprüche**
**Patentansprüche für folgende Vertragsstaaten : AT, BE, CH, DE, DK, FR, GB, IT, LI, LU, NL, SE**

1. Verbindung der Formel I

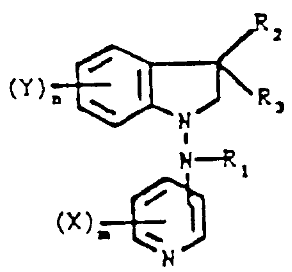

(I)

in welcher $R_1$ für Wasserstoff, $C_1$-$C_6$-Alkyl, Alkenyl oder Alkynyl steht, $R_2$ und $R_3$ unabhängig voneinander Wasserstoff oder $C_1$-$C_6$-Alkyl darstellen oder zusammen ein Spiro(benzocycloalkan) bilden; X für Halogen, $C_1$-$C_6$-Alkyl oder Nitro steht, und Y für $C_1$-$C_6$-Alkyl steht, m und n unabhängig voneinander die ganzen Zahlen 0 oder 1 sind; ihre pharmazeutisch verträglichen Säureadditionssalze und gegebenenfalls ihre optischen, geometrischen und Stereoisomere und racemischen Gemische.

2. Verbindung gemäß Anspruch 1, in welcher $R_1$ für Wasserstoff oder $C_1$-$C_6$-Alkyl steht, $R_2$ für Wasserstoff oder $C_1$-$C_6$-Alkyl steht und $R_3$ für Wasserstoff oder $C_1$-$C_6$-Alkyl steht.

3. Verbindung gemäß Anspruch 2, in welcher $R_1$ für Wasserstoff oder $C_1$-$C_6$-Alkyl steht und $R_2$ und $R_3$ Wasserstoff bedeuten.

4. Verbindung gemäß Anspruch 3, bei der es sich um 2,3-Dihydro-N-(4-pyridinyl)-1H-indol-1-amin oder ein pharmazeutisch verträgliches Säureadditionssalz davon handelt.

5. Verbindung gemäß Anspruch 3, bei der es sich um 2,3-Dihydro-N-propyl-N-(4-pyridinyl)-1H-indol-amin oder ein pharmazeutisch verträgliches Säureadditionssalz davon handelt.

6. Pharmazeutische Zusammensetzung, die als Wirkstoff eine Verbindung gemäß Anspruch 1 und eine geeignete Trägersubstanz dafür enthält.

7. Verwendung einer Verbindung gemäß Anspruch 1 zur Herstellung eines Medikaments mit analgetischer Wirksamkeit, krampflösender Wirksamkeit und/oder Wirksamkeit zur Förderung der Gedächtnisleistung.

8. Verfahren zur Herstellung einer Verbindung gemäß Anspruch 1, das das Reduzieren einer Verbindung der Formel II

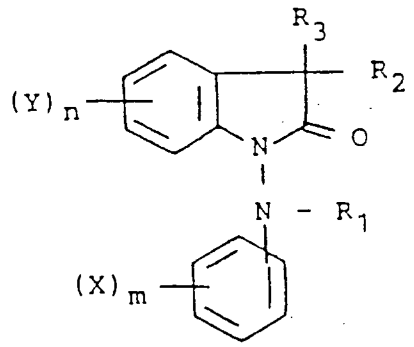

(II)

umfaßt, in welcher $R_1$, $R_2$, $R_3$, X, Y, m und n die in Anspruch 1 zugewiesene Bedeutung zukommt.

**Patentsprüche für folgende Vertragsstaaten : ES, GR**

1. Verfahren zur Herstellung einer Verbindung der Formel I

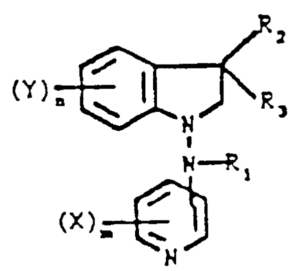

(I)

in welcher $R_1$ für Wasserstoff, $C_1$-$C_6$-Alkyl, Alkenyl oder Alkynyl steht, $R_2$ und $R_3$ unabhängig voneinander Wasserstoff oder $C_1$-$C_6$-Alkyl darstellen oder zusammen ein Spiro(benzocycloalkan)bilden; X für Halogen, $C_1$-$C_6$-Alkyl oder Nitro steht, und Y für $C_1$-$C_6$-Alkyl steht, m und n unabhängig voneinander die ganzen Zahlen 0 oder 1 sind; ihre pharmazeutisch verträglichen Säureadditionssalze und gegebenenfalls ihre optischen, geometrischen und Stereoisomere und racemischen Gemische; das das Reduzieren einer Verbindung der Formel II

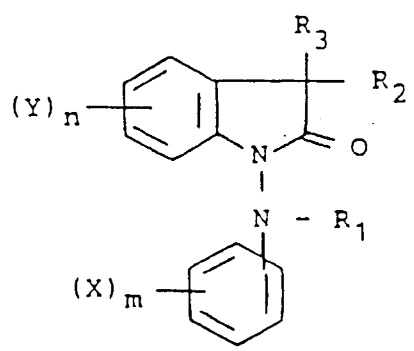

(II)

umfaßt, in welcher $R_1$, $R_2$, $R_3$, X, Y, m und n die vorstehend zugewiesene Bedeutung zukommt.

2. Verfahren gemäß Anspruch 1, wobei die Reduktion mit einem Boran-tetrahydrofurankomplex oder Lithiumaluminiumhydrid durchgeführt wird.

3. Verfahren gemäß Anspruch 1, wobei $R_1$ für Wasserstoff oder $C_1$-$C_6$-Alkyl steht, $R_2$ für Wasserstoff oder $C_1$-$C_6$-Alkyl steht und $R_3$ für Wasserstoff oder $C_1$-$C_6$-Alkyl steht.

**4.** Verfahren gemäß Anspruch 1, wobei $R_1$ für Wasserstoff oder $C_1$-$C_6$-Alkyl steht und $R_2$ und $R_3$ Wasserstoff bedeuten.

**5.** Verfahren gemäß Anspruch 1, bei dem 2,3-Dihydro-N-(4-pyridinyl)-1H-indol-1-amin oder ein pharmazeutisch verträgliches Säureadditionssalz davon hergestellt wird.

**6.** Verfahren gemäß Anspruch 1, bei dem 2,3-Dihydro-N-propyl-N-(4-pyridinyl)-1H-indol-amin oder ein pharmazeutisch verträgliches Säureadditionssalz davon hergestellt wird.

**7.** Verwendung einer Verbindung gemäß Anspruch 1 zur Herstellung eines Medikaments mit analgetischer Wirksamkeit, krampflösender Wirksamkeit und/oder Wirksamkeit zur Förderung der Gedächtnisleistung.

**Revendications**

**Revendications pour les Etats contractants suivants : AT, BE, CH, DE, DK, FR, GB, IT, LI, LU, NL, SE**

**1.** Composé de formule I

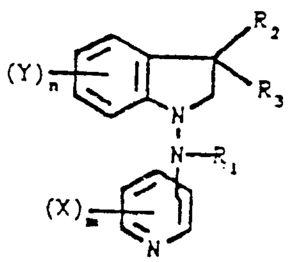

(I)

dans laquelle $R_1$ est un atome d'hydrogène ou un groupe alkyle en $C_1$-$C_6$, alcényle ou alcynyle; $R_2$ et $R_3$ sont indépendamment un atome d'hydrogène ou un groupe alkyle en $C_1$-$C_6$ ou forment ensemble un groupe spiro(benzocycloalcane) ;
X est un atome d'halogène ou un groupe alkyle en $C_1$-$C_6$ ou nitro et Y est un groupe alkyle en $C_1$-$C_6$; m et n sont indépendamment le nombre entier 0 ou 1, sels d'addition avec des acides pharmaceutiquement acceptables de ce composé et, lorsque cela est applicable, isomères optiques, géométriques et stéréoisomères et mélanges racémiques de celui-ci.

**2.** Composé selon la revendication 1, dans lequel $R_1$ est un atome d'hydrogène ou un groupe alkyle en $C_1$-$C_6$, $R_2$ est un atome d'hydrogène ou un groupe alkyle en $C_1$-$C_6$ et $R_3$ est un atome d'hydrogène ou un groupe alkyle en $C_1$-$C_6$.

**3.** Composé selon la revendication 2, dans lequel $R_1$ est un atome d'hydrogène ou un groupe alkyle en $C_1$-$C_6$, et $R_2$ et $R_3$ sont des atomes d'hydrogène.

**4.** Composé selon la revendication 3, qui est la 2,3-dihydro-N-(4-pyridinyl)-1H-indol-1-amine ou un sel d'addition avec un acide pharmaceutiquement acceptable de celle-ci.

**5.** Composé selon la revendication 3, qui est la 2,3-dihydro-N-propyl-N-(4-pyridinyl)-1H-indol-1-amine ou un sel d'addition avec un acide pharmaceutiquement acceptable de celle-ci.

**6.** Composition pharmaceutique comprenant en tant que composant actif un composé selon la revendication 1, et un véhicule approprié pour celui-ci.

**7.** Utilisation d'un composé selon la revendication 1, pour la fabrication d'un médicament ayant une activité analgésique, anticonvulsivante et/ou d'amélioration de la mémoire.

**8.** Procédé pour la préparation d'un composé tel que défini dans la revendication 1, comprenant la réduction d'un composé de formule II

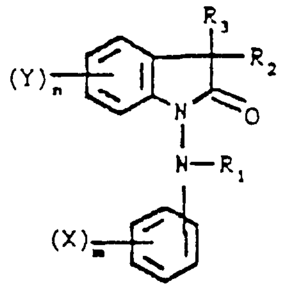

(II)

dans laquelle $R_1$, $R_2$, $R_3$, X, Y, m et n sont tels que définis dans la revendication 1.

**Revendications pour les Etats contractants suivants : ES, GR**

1. Procédé pour la préparation d'un composé de formule I

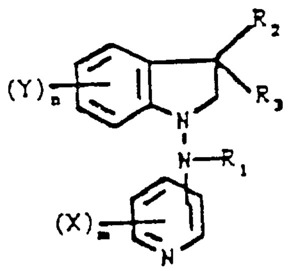

(I)

dans laquelle $R_1$ est un atome d'hydrogène ou un groupe alkyle en $C_1$-$C_6$, alcényle ou alcynyle; $R_2$ et $R_3$ sont indépendamment un atome d'hydrogène ou un groupe alkyle en $C_1$-$C_6$ ou forment ensemble un groupe spiro(benzocycloalcane); X est un atome d'halogène ou un groupe alkyle en $C_1$-$C_6$ ou nitro et Y est un groupe alkyle en $C_1$-$C_6$; m et n sont indépendamment le nombre entier 0 ou 1, de ses sels d'addition avec des acides pharmaceutiquement acceptables et, lorsque cela est applicable, de ses isomères optiques, géométriques et stéréoisomères et mélanges racémiques, comprenant la réduction d'un composé de formule II

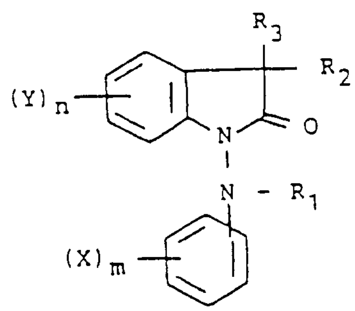

(II)

dans laquelle $R_1$, $R_2$, $R_3$, X, Y, m et n sont tels que définis précédemment.

2. Procédé selon la revendication 1, dans lequel la réduction est effectuée à l'aide d'hydrure de lithium et d'aluminium ou d'un complexe borane-tétrahydrofuranne.

3. Procédé selon la revendication 1, dans lequel $R_1$ est un atome d'hydrogène ou un groupe alkyle en $C_1$-$C_6$, $R_2$ est un atome d'hydrogène ou un groupe alkyle en $C_1$-$C_6$ et $R_3$ est un atome d'hydrogène ou un groupe alkyle en $C_1$-$C_6$.

4. Procédé selon la revendication 1, dans lequel $R_1$ est un atome d'hydrogène ou un groupe alkyle en $C_1$-$C_6$, et $R_2$ et $R_3$ sont des atomes d'hydrogène.

**5.** Procédé selon la revendication 1, dans lequel on prépare la 2,3-dihydro-N-(4-pyridinyl)-1H-indol-1-amine ou un sel d'addition avec un acide pharmaceutiquement acceptable de celle-ci.

**6.** Procédé selon la revendication 1, dans lequel on prépare la 2,3-dihydro-N-propyl-N-(4-pyridinyl)-1H-indol-1-amineou un sel d'addition avec un acide pharmaceutiquement acceptable de celle-ci.

**7.** Utilisation d'un composé tel que défini dans la revendication 1, pour la fabrication d'un médicament ayant une activité analgésique, anticonvulsivante et/ou d'amélioration de la mémoire.